# EUROPÄISCHE PATENTSCHRIFT

(11) **EP 4 021 735 B1**
(45) Veröffentlichungstag und Bekanntmachung des Hinweises auf die Patenterteilung: **12.02.2025**
(21) Anmeldenummer: 20750258.4
(22) Anmeldetag: 31.07.2020
(51) Int. Cl.: B42D 25/23, B42D 25/28, B42D 25/455, B42D 25/46, B42D 13/00, B41M 3/14, B41M 5/00, G01N 33/34, G01N 3/56, G01N 3/08

(54) **VERFAHREN ZUR PRÜFUNG DER BESTÄNDIGKEIT VON DATENTRÄGERN UND SICHERHEITSDOKUMENTEN**
METHOD FOR TESTING THE RESISTANCE OF DATA CARRIERS AND SECURITY DOCUMENTS
PROCÉDÉ POUR CONTRÔLER LA RÉSISTANCE DE SUPPORTS DE DONNÉES ET DE DOCUMENTS DE SÉCURITÉ

(30) Priorität: 29.08.2019 DE 102019123227
(43) Veröffentlichungstag der Anmeldung: 06.07.2022
(73) Patentinhaber: Bundesdruckerei GmbH, 10969 Berlin (DE)
(72) Erfinder: WÖLKI, Stephan, 12101 Berlin (DE); LESCHE, Ulrike, 13156 Berlin (DE); DITTRICH, Bettina, 12167 Berlin (DE)
(74) Vertreter: Mammel und Maser Patentanwälte PartG mbB
(86) Internationale Anmeldenummer: PCT/EP2020/071703
(87) Internationale Veröffentlichungsnummer: WO 2021/037480

(56) Entgegenhaltungen:
- EP-A2- 1 770 385
- AU-A4- 2017 100 680
- DE-A1- 102015 208 802
- US-B2- 8 329 777

## Beschreibung

Die vorliegende Erfindung betrifft ein Verfahren zur Prüfung der Beständigkeit von Datenträgern wie ID-, Wert- oder Ausweisdokumenten und Sicherheitsdokumenten. Insbesondere betrifft die Erfindung ein Prüfverfahren für die Beständigkeit von Datenträgern und Sicherheitsdokumenten, wie beispielsweise Ausweisen, Firmenausweisen etc., die durch Bedruckung und/oder andere Sicherheitsmerkmale personalisiert sind, gegenüber Desinfektionsmitteln, insbesondere im medizinischen Umfeld.

Bei Sicherheitsdokumenten ist entscheidend, dass trotz intensiver Nutzung während der gesamten Nutzungsdauer keine oder nur geringe Änderungen im Aussehen und in der Funktionalität erkennbar sein dürfen. Dies gilt insbesondere für die Personalisierung.

Datenträger und Sicherheitsdokumente werden häufig aus verschiedenen Schichten mit Sicherheitsmerkmalen und unter Einbringung der Personalisierung durch Laminieren hergestellt, wobei die Personalisierung und die Sicherheitsmerkmale grundsätzlich in dem Sicherheitsdokument oder Datenträger und/oder an dessen Oberfläche eingebracht sein können.

Die EP 1770385 A2 beschreibt ein Verfahren zur Überprüfung der Beständigkeit von Sicherheitsdokumenten und Datenträgern, wobei auf der Oberfläche von Sicherheitsdokumenten oder Datenträgern mit einem Reibepad das zuvor mit einer künstlichen Schweißlösung als Testflüssigkeit benetzt wurde, gescheuert wird.

Die DE 10 2015 208 802 A1 lehrt ein Verfahren zum Untersuchen der Beständigkeit von Materialien, die zur Herstellung von Wert- und Sicherheitsprodukten verwendet werden, gegen Veränderungen von deren Eigenschaften bei deren Gebrauch.

Die AU 2017 100 680 A4 lehrt ein Verfahren zur Bestimmung der Scheuerbeständigkeit eines funktionalisierten Substrats zur Herstellung oder zur Benutzung als Sicherheitsdokument, wobei die Scheuersimulation einen mechanischen Stresstest, einen chemischen Stresstest bei einer Temperatur von wenigstens 50 °C und einen mechanischen Fehlertest umfasst.

Sicherheitsdokumente, wie Ärzteausweise und Krankenkarten, werden im medizinischen Umfeld eingesetzt, wo sie in Kontakt mit Desinfektionsmitteln kommen können. Häufig wird bei diesen Sicherheitsdokumenten die Personalisierung mittels Thermotransfer- oder Thermosublimationsverfahren auf die Oberfläche aufgedruckt oder es wird eine spezielle Inkjet-Tinte eingesetzt, die in die oberste Schicht hineindiffundiert. Durch den Kontakt mit den Desinfektionsmitteln kann das Dokument beschädigt und die Lesbarkeit der Personalisierung und/oder das Sicherheitsmerkmal beeinträchtigt werden.

Aus der US 8,329,777 B2 ist ein Verfahren zur Überprüfung der Beständigkeit von Sicherheitsdokumenten bekannt, bei dem auf der Oberfläche des Sicherheitsdokuments mit auf Wattestäbchen appliziertem Isopropylacetat oder Ethanol gescheuert wird.

Hiervon ausgehend ist die Aufgabe der vorliegenden Erfindung, ein Verfahren anzugeben, mit dem die Beeinträchtigung von Sicherheitsdokumenten oder Datenträgern durch Desinfektionsmittel, insbesondere im medizinischen Umfeld, auf einfache Weise geprüft und realistische Bedingungen der Beeinträchtigung auf einfache Weise nachgestellt werden können.

Diese Aufgabe wird durch die Merkmale des Anspruchs 1 gelöst.

Bei dem erfindungsgemäßen Verfahren wird die Beständigkeit von Dokumenten gegenüber Desinfektionsmitteln dadurch bestimmt, dass auf der Oberfläche von Sicherheitsdokumenten oder Datenträgern mit Textilstücken, die zuvor mit einer Testflüssigkeit benetzt werden, unter definierten Bedingungen gescheuert wird, wobei der Scheuertest mit wenigstens zwei unterschiedlichen Testflüssigkeiten durchgeführt wird und die wenigstens zwei unterschiedlichen Testflüssigkeiten aus den Gruppen der wässrigen alkoholischen Lösungen, der hydrophobe Verbindungen enthaltenden Flüssigkeiten und der Biozide enthaltenden Lösungen ausgewählt werden.

Durch die Durchführung der Tests mit den wenigstens zwei verschiedenen Testflüssigkeiten aus den definierten Gruppen der Testflüssigkeiten können gute Ergebnisse über die Beständigkeit der Sicherheitsdokumente gegenüber den Desinfektionsmitteln im medizinischen Umfeld, beispielsweise im Rahmen der Händedesinfektion, bei einer geringen Anzahl von Versuchen erzielt werden, ohne dass der Scheuertest auf der Oberfläche des Sicherheitsdokuments mit einer Vielzahl der sich auf dem Markt befindenden unterschiedlichen Desinfektionsmitteln durchgeführt werden muss.

Im Rahmen der vorliegenden Erfindung wurde festgestellt, dass realistische Beeinträchtigungen dadurch simuliert werden können, dass der Scheuertest mit einem flächigen Textil durchgeführt wird. Es versteht sich, dass für einen jeden Scheuertest mit einer Testflüssigkeit ein neues flächiges Textil verwendet wird.

Das erfindungsgemäße Verfahren ermöglicht es, die mechanischen und chemischen Belastungen durch Desinfektionsmittel im medizinischen Umfeld auf einfache Weise zu simulieren und die Langzeiteignung verschiedener Oberflächen von Sicherheitsdokumenten oder Datenträgern und die Haltbarkeit der Personalisierung, der Sicherheitsmerkmale, Drucke, Applikationen etc. gegenüber Desinfektionsmittel im medizinischen Umfeld zu überprüfen und abzusichern.

Erfindungsgemäß werden die Scheuertests mit wenigstens zwei unterschiedlichen Testflüssigkeiten aus den nachfolgend erläuterten drei Gruppen von Testflüssigkeiten durchgeführt.

Eine Testflüssigkeit ist eine wässrige alkoholische Lösung (nachfolgend erste Testflüssigkeit). Der Alkoholanteil in der Lösung sollte maximal 96 Gew.% und vorzugsweise zwischen 50 Gew.% und 95 Gew.% betragen und besonders bevorzugt zwischen 60 Gew.% und 90 Gew.%.

Grundsätzlich können in der ersten Testflüssigkeit alle üblichen Alkohole, insbesondere Dialkohole und Trialkohole, vorzugsweise mit zwischen 1 und 14 Kohlenstoffatomen, eingesetzt werden. Besonders bevorzugte Alkohole sind Ethanol, n-Propanol, Propan-2-ol, n-, iso- oder tert.-Butanol und Butan-1,3-diol.

Zudem kann in dieser ersten Testflüssigkeit auch ein Gemisch von verschiedenen Alkoholen, wie beispielsweise Ethanol/ Propan-2-ol, Ethanol/Butanol oder Ethanol/Pentan-1-ol, Ethanol/Propan-1,2-diol, Ethanol/Butan-1,3-diol, Ethanol/Propan-1,2,3-triol, Propan-1-ol/Propan-2-ol eingesetzt werden.

In einer ersten bevorzugten Ausführungsform ist die erste Testflüssigkeit eine konzentrierte wässrige Ethanollösung, in einer weiteren bevorzugten Ausführungsform ist die erste Testflüssigkeit eine konzentrierte wässrige Isopropanol-Lösung.

Im Rahmen der Erfindung ist es auch möglich, die Scheuertests nacheinander mit beiden bevorzugten Varianten der ersten Testflüssigkeit durchzuführen, somit mit einer Ethanollösung und einer Isopropanollösung.

Eine weitere Testflüssigkeit, die nachfolgend als "zweite Testflüssigkeit" bezeichnet wird, ist eine hydrophobe Verbindungen enthaltende Flüssigkeit, wobei die hydrophoben Verbindungen, vorzugsweise Kohlenwasserstoffe, insbesondere Alkane oder ein Gemisch von Alkanen sind. Der Anteil an hydrophoben Verbindungen, wie Kohlenwasserstoffen, insbesondere Alkanen, in der Flüssigkeit beträgt vorzugsweise zwischen 2 Gew.% und 30 Gew.% und besonders bevorzugt zwischen 3 Gew.% und 10 Gew.%. Grundsätzlich können für die zweite Testflüssigkeit alle flüssigen, vorzugsweise leicht flüchtigen, Alkane ausgewählt werden, insbesondere Alkane mit einer C-Kettenlänge zwischen 5 und 9 Kohlenstoffatomen. Besonders bevorzugt ist der Einsatz von Petrolether (Petroletherbenzin) in einem Siedebereich von 30 °C - 70 °C, bzw. 40 °C - 65 °C oder Ligroin mit einem Siedebereich von 60 °C bis 100 °C .

Im Allgemeinen ist die hydrophobe Komponente in dieser zweiten Testflüssigkeit in einem Lösemittel wie einem Alkohol gelöst. Der Lösemittelanteil liegt bei der zweiten Testflüssigkeit vorzugsweise in einem Konzentrationsbereich zwischen 70 Gew.% und 98 Gew.%, besonders bevorzugt zwischen 90 Gew.% und 97 Gew.%. Vorzugsweise ist das Lösemittel ein Alkohol aus der Gruppe Ethanol, Propan-1-ol, Propan-2-ol oder einem Butanol oder Butandiol.

Die dritte Testflüssigkeit enthält Biozide, beispielsweise quartäre Ammoniumverbindungen, wie beispielsweise Trimethyltetradecylammoniumbromid, Benzalkoniumchlorid, Cetylalkoniumchlorid, Cetylpyridiniumchlorid, Tetrabutylammoniumhydroxid, Paraquat Polyquaternium-7 oder Tetradecyltrimethylammoniumoxalat oder Biphenylol.

Die Biozidkonzentration liegt im Allgemeinen zwischen 0,02 Gew.% und 5 Gew.%, vorzugsweise von 0,5 Gew.% bis 4 Gew.% und besonders bevorzugt 1 Gew.% bis 3 Gew.%.

Das oder die Biozide können in einem hydrophilen Lösemittel oder einem hydrophilen Lösemittelgemisch, beispielsweise Wasser oder Alkohol, insbesondere Ethanol, gelöst sein.

Alle Testflüssigkeiten sind vorzugsweise Lösungen.

Unter einem Textilstück werden im Rahmen der vorliegenden Erfindung Rohstoffe verstanden, die zu flächenförmigen Gebilden wie Geweben, Gewirken, Gestricken, Geflechten, Nähgewirken, Vliesstoffen und Filzen einer begrenzten Ausdehnung verarbeitet sind. Vorzugsweise ist das flächige Textil aus Baumwolle, besonders bevorzugt ist es ein Baumwollgewebe, vergleichbar mit "Cotton Drill 702-480", das für die Norm ISO 105-D01 eingesetzt wird.

Durch den Einsatz der Testflüssigkeiten kann einerseits eine verlässliche Aussage über die Beständigkeit von Sicherheitsdokumenten gegenüber Desinfektionsmitteln getroffen werden, zudem lassen sich die Ergebnisse aufgrund der Verwendung der definierten Testflüssigkeiten und der definierten Testbedingungen normieren, sodass definierte langjährig reproduzierbare Prüfbedingungen geschaffen werden.

Dies ist beim Einsatz von handelsüblichen Desinfektionsmitteln nicht möglich, da häufig eine langjährig konstante chemische Zusammensetzung nicht gegeben ist.

Die Beständigkeit des Sicherheitsdokuments wird mit wenigstens zwei, vorzugsweise mit drei oder mehr verschiedenen Testflüssigkeiten aus den Gruppen der erfindungsgemäßen Testflüssigkeiten unter definierten Bedingungen geprüft. Hierbei werden die Testflüssigkeiten bei zwei Testflüssigkeiten vorzugsweise so ausgewählt, dass sie nicht derselben Gruppe von Testflüssigkeiten angehören.

Wird der Test mit drei Testflüssigkeiten durchgeführt, so sind wenigstens zwei der Testflüssigkeiten vorzugsweise aus unterschiedlichen Gruppen von Testflüssigkeiten.

Wenn zwei oder mehr Testflüssigkeiten in einer Variante eingesetzt werden, können auch zwei verschiedene Testflüssigkeiten aus der Gruppe der wässrigen alkoholischen Lösungen ausgewählt werden.

In einer anderen Variante werden alle drei Testflüssigkeiten aus unterschiedlichen Gruppen ausgewählt.

Wird der Scheuertest mit vier verschiedenen Testflüssigkeiten durchgeführt, so sind bevorzugt zwei Testflüssigkeiten aus der ersten Gruppe der wässrigen alkoholischen Lösungen und jeweils eine Testflüssigkeit aus der zweiten und dritten Gruppe auszuwählen.

Für jede Testflüssigkeit wird dann der Einzeltest mit einem mit der jeweiligen Testflüssigkeit benetzten Stück, vorzugsweise Baumwollstück, durchgeführt, wobei mit dem benetzten Textilstück ein Scheuertest auf der Dokumentenoberfläche mit einer den Gebrauchserwartungen entsprechendem Scheuern oder Reiben durchgeführt wird.

Unter "Benetzen" wird im Rahmen der Erfindung verstanden, dass das Textilstück in der Testflüssigkeit getränkt oder mit der Testflüssigkeit besprüht oder beträufelt wird.

Wesentlich ist, dass im Rahmen der vorliegenden Erfindung die betreffende Anzahl von verschiedenen Testflüssigkeiten aus den verschiedenen Gruppen grundsätzlich frei miteinander kombiniert werden können, beispielsweise eine 80%ige Propan-2-ol/Ethanollösung in Wasser als erste Testflüssigkeit, 10 % Octan in 90 % Butanollösung als zweite Testflüssigkeit und eine Lösung von 4 Gew.% Tetrabutylammoniumhydroxid in Isopropanol als dritte Testflüssigkeit.

Unter Scheuern wird im Rahmen der Erfindung eine Translations- oder Rotationsbewegung entlang der Oberfläche des Dokuments unter einem gewissen Druck mit einer bestimmten Fläche verstanden. Ein Scheuertest wird auch als Abriebtest bezeichnet.

Das Scheuern kann durch eine festgelegte Anzahl von Doppelhüben unter Einwirkung eines definierten Scheuergewichts entlang einer geraden Linie erfolgen, durch eine belastende Drehbewegung oder durch eine Zickzack-Bewegung mit definierten Winkeln zueinander.

Unter dem Scheuern "unter definierten Bedingungen" wird verstanden, dass der Test jeweils für die gewünschte Anzahl von Testlösungen unter denselben Bedingungen wie Scheuerdruck, Scheuerfläche, Menge an benetztem Lösemittel, Textilstück, Scheuerweg, Anzahl der Doppelhöhe etc. wiederholt wird.

Damit wird die Kombination aus mechanischer Scheuer- und chemischer Belastung im realen Einsatz simuliert. In einer bevorzugten Variante wird neben dem dem Scheuertest unterzogenen Sicherheitsdokument nach dem Scheuertest auch das Textilstück untersucht, beispielsweise ob und in welchem Umfang Farbe von dem Sicherheitsdokument auf das Textilstück abgefärbt hat oder - anders ausgedrückt - ob und inwieweit das Dokument das Desinfektionsmittel angreifbar, manipulierbar und veränderbar macht.

Dieses Verfahren ist generell für alle Arten von Dokumenten (auch Papierdokumenten) und sich im Handel befindenden Desinfektionsmitteln für das medizinische Umfeld geeignet.

Falls gewünscht, kann der Scheuertest zusätzlich noch mit dem jeweils gewünschten Desinfektionsmittel und/oder dem trockenen Textilstück durchgeführt werden.

In einer weiteren Variante werden zur Überprüfung der Beständigkeit gegenüber Extrembelastungen durch Desinfektionsmittel die Sicherheitsdokumente zusätzlich komplett in die jeweilige Testflüssigkeit eingetaucht und anschließend mit einem trockenen Textil oder einem mit der jeweiligen Testflüssigkeit benetzten Textil unter definierten Bedingungen gescheuert. Hierbei ist die gewählte Einwirkdauer und die gewählte Scheuerdauer so festzulegen, dass sie wenigstens den angenommenen Extrembelastungen entsprechen. In einer bevorzugten Variante eines solchen zusätzlichen Tests auf Extrembelastung wurde die Einwirkdauer mit fünf Minuten festgelegt und damit mehr als doppelt so lange, wie für die Händedesinfektion (0,5 Minuten bis 2 Minuten) nach dem Bundesgesundheitsblatt festgelegt.

Das trockene Textilgewebe ist häufig je nach Materialart unbenetzt an der Oberfläche härter. Die Belastung für die Probe im Scheuertest ist dadurch noch intensiver.

Mit dem erfindungsgemäßen Verfahren kann nicht nur die Langzeiteignung der Qualität der jeweiligen Sicherheitsdokumente abgesichert werden, sondern auch das Reklamationsrisiko der Sicherheitsdokumente vermindert werden.

Das Verfahren ist zur Untersuchung der Beständigkeit, insbesondere von Kunststoff- oder Papieroberflächen von Sicherheitsdokumenten und Datenträgern, besonders von Polycarbonat-, PVC- oder PET-Oberflächen, geeignet.

Zudem kann die Haltbarkeit einer Personalisierung unter definierten Bedingungen geprüft werden.

Das erfindungsgemäße Verfahren kann auch mit viskoseren Flüssigkeiten durchgeführt werden, solange diese das flächige Textil benetzen können.

Die Auswertung der Scheuertests kann beispielsweise durch visuelle Kontrolle, Fotodokumentation oder UV/VIS-Spektroskopie, Fluoreszenzspektroskopie, Laserscanningmikroskopie der Oberfläche der Sicherheitsdokumente oder Datenträger erfolgen. Weiterhin kann selbstverständlich auch die Funktion etwaiger auf der Oberfläche angeordneter Chips oder einlaminierter Chips mit entsprechend verknüpfter Antenne überprüft werden. Eine weitere Auswertung der Beeinträchtigung der Oberfläche des Sicherheitsdokuments oder des Datenträgers kann durch Untersuchung mit Speziallichtarten, Ultraschallmikroskopie und diverser elektronischer Auslese-Tests erfolgen.

Weiterhin können nach der Durchführung der Scheuertests auch die eingesetzten flächigen Textilstücke ausgewertet werden, beispielsweise durch spektroskopische Analyse der Art und Menge des herausgelösten Farbstoffs, Analytik des Farbstoffs, Detektion von Metall- oder Halbleiterspuren, die herausgescheuert wurden. Über eine digitale Aufnahme mit Pixelauswertung über Graustufen kann die Menge des Abriebs quantitativ analysiert werden.

Die Fläche des Textilstücks, mit dem gescheuert wird, liegt im Allgemeinen zwischen 45 bis 55 cm².

Der Anpressdruck ist an die zu simulierenden Gegebenheiten anzupassen und beträgt vorzugsweise zwischen 0,3 N/cm² und 0,7 N/cm².

Das Scheuern unter definierten Bedingungen erfolgt vorzugsweise in einem Scheuerprüfgerät.

Die Erfindung betrifft auch ein in Anspruch 10 definiertes Set von Testflüssigkeiten.

Die Erfindung ist nachfolgend anhand eines Ausführungsbeispiels näher beschrieben.

### Ausführungsbeispiel

Zur Überprüfung der Beständigkeit eines Sicherheitsdokuments mit einer Oberfläche aus Polycarbonat, PVC, PET und EVA gegenüber den Desinfektionsmitteln wurden die folgenden Testflüssigkeiten eingesetzt:

### Gruppe der eingesetzten Testflüssigkeiten:

1. Testflüssigkeit:
   a) 80 Gew.% wässrige Lösung Ethanol (reinst, 96 % Mindestgehalt)* alternativ oder zusätzlich zu a)
   b) 70 Gew.-% wässrige Lösung Isopropanol (reinst, 96 % Mindestgehalt)*
2. Testflüssigkeit:
   95 Gew.% wässrige Lösung Ethanol (reinst, 96 % Mindestgehalt)
   + 5 Gew.% Petroleumbenzin 40 - 65*
3. Testflüssigkeit:
   98 Gew.% Ethanol (reinst, 96 % Mindestgehalt) + 2 Gew.%
   Trimethyletradecylammoniumbromid (Biozid) (darin gelöst)

*Die wässrigen Lösungen wurden mit entionisiertem Wasser eingesetzt.

### Vergleichsprodukt: Sterillium Handelsprodukt

C. Es wurden Sicherheitsdokumente aus verschiedenen laminierten Schichten untersucht, auf deren Oberfläche aus PET, PVC, EVAoder Polycarbonat mit Thermotransferverfahren oder Thermosublimationsverfahren eine Personalisierung aufgedruckt war oder bei denen die Personalisierung mit einer speziellen Inkjet-Tinte in die Oberfläche hineindiffundierte. Bei all diesen verschiedenen Sicherheitsdokumenten kann die Testflüssigkeit grundsätzlich den Farbstoff oder die Tinte anlösen und die Personalisierung teilweise entfernen.

### D. Scheuertest mit benetztem Baumwollstück

Zur Durchführung des Scheuertests wurde ein Scheuerprüfgerät Typ Quartant der Firma prüfbau eingesetzt. Die Geräteeigenschaften waren wie folgt:

| | |
|---|---|
| 0,5 N/cm² | Scheuerdruck, entspricht ca. 600 g Scheuergewicht |
| 40 mm | Durchmesser der Scheuerfläche (entsprechend circa 1.250 mm² |
| 5 cm | Scheuerweg |

Die Anzahl der Scheuerzyklen betrug 100 Doppelhübe. Doppelhübe beschreiben den doppelten Scheuerweg, der einmal in der Hinbewegung und dann in der Rückbewegung durchlaufen wird. Das Scheuergewicht wurde mit einem Baumwollgewebe als flächigem Textilstück versehen, das zuvor jeweils mit der zu untersuchenden Testflüssigkeit benetzt wurde.

Die Scheuertests mit den vier verschiedenen Testflüssigkeiten wurden auf der Oberfläche von vier identischen Sicherheitsdokumenten durchgeführt, wobei jeweils ein Baumwollgewebe mit einer der obigen vier Testflüssigkeiten 1a, 1b, 2 und 3 benetzt wurde.

Die personalisierte Oberfläche eines jeden Sicherheitsdokuments wurde dabei auf der Vorderseite mit einem Anpressdruck von 0,5 N/cm² und einer runden Scheuerfläche mit einem Durchmesser von 40 mm mit 100 Doppelhüben getestet.

### E. Zusätzlicher Test unter Extrembedingungen:

Scheuertest nach Eintauchen in Testflüssigkeit Um zusätzlich Extrembedingungen zu simulieren, wurde jeweils ein Sicherheitsdokument mit einer Einwirkdauer von fünf Minuten vollständig in eine Testlösung eingetaucht. Nach der Entnahme wurde das Dokument trockengetupft und innerhalb von einer Minute einem Scheuertest wie oben unter D. beschrieben, jedoch mit einem trockenen Baumwollgewebe, unterzogen.

### F. Auswertung

Nach jedem nach C. oder D. durchgeführten Test wurde die Lesbarkeit der Personalisierung im Vergleich zu einem unbehandelten Sicherheitsdokument visuell unter Tageslicht bewertet. Hierfür wurden Sicherheitsdokumente mit geringem und hohem Schwärzungsgrad der Personalisierung eingesetzt. Ferner erfolgte eine Prüfung auf Veränderungen des Sicherheitsdokuments auf Risse, Verformungen, Ablösungen im Verbund etc.

### G. Ergebnisse

Die Versuche mit den Testchemikalien Nr. 1a, 1b, 2, 3 und auch der Vergleichsversuch mit dem käuflich erworbenen Vergleichsprodukt " Sterilium" ergaben bei dem Scheuertest mit den benetzten Baumwollstücken und auch bei dem nachfolgenden Versuch D. unter extremeren Bedingungen mit dem Eintauchen in die Testflüssigkeit und nachfolgendem Scheuertest keine Auffälligkeiten, es waren keine oder nur sehr leichte Veränderungen der getesteten Vorderseite und deren Personalisierung sichtbar. Die eingesetzten Textilien als Scheuermedium zeigten keine Farbspuren.

## Patentansprüche

1. Verfahren zur Überprüfung der Beständigkeit von Sicherheitsdokumenten und Datenträgern gegenüber Desinfektionsmitteln, wobei auf der Oberfläche von Sicherheitsdokumenten oder Datenträgern mit flächigen Textilstücken, die zuvor mit einer Testflüssigkeit benetzt werden, unter definierten Bedingungen gescheuert wird, wobei der Scheuertest mit wenigstens zwei unterschiedlichen Testflüssigkeiten durchgeführt wird und die wenigstens zwei unterschiedlichen Testflüssigkeiten aus den Gruppen der wässrigen alkoholischen Lösungen, der hydrophobe Verbindungen enthaltenden Flüssigkeiten und der Biozide enthaltenden Lösungen ausgewählt werden.

2. Verfahren nach Anspruch 1, **dadurch gekennzeichnet, dass** als Testflüssigkeit aus der Gruppe der wässrigen alkoholischen Lösungen eine Lösung mit einem Alkoholanteil von maximal 96 Gew.%, vorzugsweise zwischen 50 Gew.% und 95 Gew.% und besonders bevorzugt zwischen 60 Gew.% und 90 Gew.% verwendet wird und/oder die Alkohole vorzugsweise zwischen 1 und 14 Kohlenstoffatomen aufweisen und besonders bevorzugt aus der Gruppe Ethanol, n-Propanol, Isopropanol, n-, iso- oder tert.-Butanol oder Butan-1,3-diol ausgewählt werden.

3. Verfahren nach Anspruch 2, **dadurch gekennzeichnet, dass** die erste Testflüssigkeit ein Gemisch von verschiedenen Alkoholen, vorzugsweise ein Ethanol, n-Propanol, Propan-2-ol, n-, iso- oder tert.-Butanol und Butan-1,3-diol -Gemisch ist.

4. Verfahren nach einem der vorangegangenen Ansprüche, **dadurch gekennzeichnet, dass** die Testflüssigkeit, die eine hydrophobe Verbindungen enthaltende Flüssigkeit ist, Kohlenwasserstoffe, insbesondere Alkane oder ein Gemisch von Alkanen enthält, besonders bevorzugt mit einer C-Kettenlänge zwischen 5 und 9 Kohlenstoffatomen, wobei der Anteil an hydrophoben Verbindungen in der Flüssigkeit vorzugsweise zwischen 2 Gew.% und 30 Gew.% und besonders bevorzugt zwischen 3 Gew.% und 10 Gew.% beträgt und/oder der Lösemittelanteil bei dieser Testflüssigkeit vorzugsweise zwischen 70 Gew.% und 98 Gew.% und besonders bevorzugt zwischen 90 Gew.% und 97 Gew.% beträgt und/oder das Lösemittel vorzugsweise ein Alkohol, insbesondere aus der Gruppe Ethanol, Propan-1-ol, Propan-2-ol, Butanol oder einem Butandiol ist.

5. Verfahren nach einem der vorangegangenen Ansprüche, **dadurch gekennzeichnet, dass** die Testflüssigkeit eine Biozide enthaltende Lösung ist, wobei die Biozide vorzugsweise aus der aus quartären Ammoniumverbindungen, wie beispielsweise Trimethyltetradecylammoniumbromid, Benzalkoniumchlorid, Cetylalkoniumchlorid, Cetylpyridiniumchlorid, Tetrabutylammoniumhydroxid, Paraquat Polyquaternium-7 Tetradecyltrimethylammoniumoxalat oder den Biphenylolen ausgewählt werden, wobei die Biozidkonzentration im Allgemeinen zwischen 0,02 Gew.% und 5 Gew.%, vorzugsweise zwischen 0,5 Gew.% und 4 Gew.% und besonders bevorzugt 1 Gew.% bis 3 Gew.% beträgt und/oder das Lösemittel ein hydrophiles Lösemittel oder ein hydrophiles Lösemittelgemisch, wie Wasser oder ein Alkohol, insbesondere Ethanol, ist.

6. Verfahren nach einem der Ansprüche 1 bis 5, **dadurch gekennzeichnet, dass** der Scheuertest mit wenigstens drei unterschiedlichen Testflüssigkeiten aus den oben definierten Gruppen durchgeführt wird und die wenigstens drei unterschiedlichen Testflüssigkeiten vorzugsweise zu drei unterschiedlichen Gruppen von Testflüssigkeiten gehören und/oder zwei Testflüssigkeiten aus der Gruppe der wässrigen alkoholischen Lösungen sind.

7. Verfahren nach Anspruch 1 bis 5, **dadurch gekennzeichnet, dass** wenigstens zwei oder drei unterschiedliche Testflüssigkeiten zu unterschiedlichen Gruppen von Testflüssigkeiten gehören.

8. Verfahren nach einem der vorangegangenen Ansprüche, **dadurch gekennzeichnet, dass** das flächige Textil ein flächenförmiges Gebilde wie ein Gewebe, Gewirke, Gestrick, Geflecht, Nähgewirke, Vliesstoff und Filz einer begrenzten Ausdehnung und insbesondere ein Baumwollgewebe ist.

9. Verfahren nach einem der vorangegangenen Ansprüche, **dadurch gekennzeichnet, dass** die Sicherheitsdokumente oder Datenträger durch Bedruckung der Oberfläche mittels Thermotransfer- oder Thermosublimationsverfahren oder durch Verwendung einer speziellen Inkjet-Tinte, die in die obere Schicht hineindiffundiert, personalisiert sind und oder die Oberfläche des Sicherheitsdokuments oder Datenträgers aus Kunststoff, Papier, und insbesondere aus Polycarbonat, PET, EVA oder PVC ist.

10. Set von Testflüssigkeiten zur Durchführung der Überprüfung der Beständigkeit von Sicherheitsdokumenten und Datenträger gegenüber Desinfektionsmitteln in einem Verfahren nach einem der Ansprüche 1 bis 9, wobei das Set aus wenigstens zwei unterschiedlichen Testflüssigkeiten aus den Gruppen der wässrigen alkoholischen Lösungen, der hydrophobe Verbindungen enthaltenden Flüssigkeiten und der Biozide enthaltenden Lösungen besteht.

## Claims

1. Method for testing the resistance of security documents and data carriers to disinfectants, wherein the surface of security documents or data carriers is rubbed with flat pieces of textile which have previously been wetted with a test liquid, under defined conditions, the abrasion test being carried out with at least two different test liquids and the at least two different test liquids being selected from the groups of aqueous alcoholic solutions, liquids containing hydrophobic compounds and solutions containing biocides.

2. Method according to claim 1, **characterized in that** as test liquid from the group of aqueous alcoholic solutions is used a solution with an alcohol content of at most 96% by weight, preferably between 50% by weight and 95% by weight and particularly preferably between 60% by weight and 90 wt.% and/or the alcohols preferably have between 1 and 14 carbon atoms and are particularly preferably selected from the group consisting of ethanol, n-propanol, isopropanol, n-, iso- or tert-butanol or butane-1,3-diol.

3. Method according to claim 2, **characterized in that** the first test liquid is a mixture of different alcohols, preferably an ethanol, n-propanol, propan-2-ol, n-, iso- or tert-butanol and butane-1,3-diol mixture.

4. A method according to any of the preceding claims, **characterized in that** the test liquid, which is a liquid containing hydrophobic compounds, contains hydrocarbons, in particular alkanes or a mixture of alkanes, preferably having a carbon chain length between 5 and 9 carbon atoms, the proportion of hydrophobic compounds in the liquid preferably being between 2% by weight and 30% by weight and more preferably between 3% by weight and 10% by weight and/or the proportion of solvent in this test liquid preferably being between 70% by weight and 98% by weight and is particularly preferred to be between 90% by weight and 97% by weight and/or the solvent is preferably an alcohol, in particular from the group ethanol, propan-1-ol, propan-2-ol, butanol or a butanediol.

5. A method according to any of the preceding claims, **characterized in that** the test liquid is a solution containing biocides, the biocides preferably being selected from the group consisting of quaternary ammonium compounds, such as, for example, trimethyltetradecylammonium bromide, benzalkonium chloride, ethylalkonium chloride, cetylpyridinium chloride, tetrabutylammonium hydroxide, paraquat polyquaternium-7, tetradecyltrimethylammonium oxalate or the biphenylols, the biocide concentration generally being between 0.02% by weight and 5 wt.%, preferably between 0.5 wt.% and 4 wt.%, and most preferably 1 wt.% to 3 wt.%, and/or the solvent is a hydrophilic solvent or a hydrophilic solvent mixture, such as water or an alcohol, in particular ethanol.

6. A method according to any one of claims 1 to 5, **characterized in that** the abrasion test is carried out with at least three different test liquids from the groups defined above and the at least three different test liquids preferably belong to three different groups of test liquids and/or two test liquids are from the group of aqueous alcoholic solutions.

7. Method according to claims 1 to 5, **characterized in that** at least two or three different test liquids belong to different groups of test liquids.

8. A method according to one of the preceding claims, **characterized in that** the flat textile is a planar structure such as a woven fabric, knitted fabric, mesh, braid, stitch-bonded fabric, nonwoven fabric and felt of limited extent and in particular a cotton fabric.

9. A method according to any of the preceding claims, **characterized in that** the security documents or data carriers are personalized by printing on the surface by means of thermal transfer or thermal sublimation processes or by using a special inkjet ink that diffuses into the upper layer, and/or the surface of the security document or data carrier is made of plastic, paper, and in particular of polycarbonate, PET, EVA or PVC.

10. Set of test fluids for carrying out the test of the resistance of security documents and data carriers to disinfectants in a process according to one of claims 1 to 9, wherein the set consists of at least two different test liquids from the groups of aqueous alcoholic solutions, of liquids containing hydrophobic compounds and of solutions containing biocides.

## Revendications

1. Procédé de contrôle de la résistance de documents de sécurité et de supports de données aux agents désinfectants, dans lequel on frotte dans des conditions définies sur la surface de documents de sécurité ou de supports de données avec des pièces textiles plates qui sont préalablement mouillées avec un liquide de test, le test de frottement étant effectué avec au moins deux liquides de test différents et les au moins deux liquides de test différents étant choisis dans les groupes des solutions alcooliques aqueuses, des liquides contenant des composés hydrophobes et des solutions contenant des biocides.

2. Procédé selon la revendication 1, **caractérisé en ce que** comme liquide de test du groupe des solutions alcooliques aqueuses on utilise une solution ayant une teneur en alcool d'au plus 96 % en poids, de préférence comprise entre 50 % en poids et 95 % en poids et de manière particulièrement préférée entre 60 % en poids et 90 % en poids, et/ou **en ce que** les alcools présentent de préférence entre 1 et 14 atomes de carbone et sont choisis de manière particulièrement préférée dans le groupe constitué par l'éthanol, le n-propanol, l'isopropanol, le n-, iso- ou tert-butanol ou le butane-1,3-diol.

3. Procédé selon la revendication 2, **caractérisé en ce que** le premier liquide de test est un mélange de différents alcools, de préférence un mélange d'éthanol, de n-propanol, de propan-2-ol, de n-, iso- ou tert-butanol et de butane-1,3-diol.

4. Procédé selon l'une quelconque des revendications précédentes, **caractérisé en ce que** le liquide de test, qui est un liquide contenant des composés hydrophobes, contient des hydrocarbures, en particulier des alcanes ou un mélange d'alcanes, de manière particulièrement préférée avec une longueur de chaîne C comprise entre 5 et 9 atomes de carbone, la proportion de composés hydrophobes dans le liquide étant de préférence comprise entre 2 % et 30 % en poids et plus préférentiellement entre 3 % en poids et 10 % en poids et/ou la proportion de solvant dans ce liquide de test est de préférence comprise entre 70 % en poids et 98 % en poids et plus préférentiellement entre 90 % en poids et 97 % en poids et/ou le solvant est de préférence un alcool, notamment choisi parmi l'éthanol, le propan-1-ol, le propan-2-ol, le butanol ou un butanediol.

5. Procédé selon l'une quelconque des revendications précédentes, **caractérisé en ce que** le liquide de test est une solution contenant des biocides, lesdits biocides étant de préférence choisis dans le groupe constitué par les composés d'ammonium quaternaire, tels que le bromure de triméthyltétradécylammonium, le chlorure de benzalkonium, le chlorure de cétylalkonium, le chlorure de cétylpyridinium, l'hydroxyde de tétrabutylammonium, le paraquat polyquaternium-7 oxalate de tétradécyltriméthylammonium ou les biphénylènes, la concentration en biocide étant généralement comprise entre 0,02 % en poids et 5 % en poids, de préférence entre 0,5 % et 4 % en poids, et de manière particulièrement préférée entre 1 % et 3 % en poids, et/ou le solvant est un solvant hydrophile ou un mélange de solvants hydrophiles, comme l'eau ou un alcool, en particulier l'éthanol.

6. Procédé selon l'une quelconque des revendications 1 à 5, **caractérisé en ce que** le test d'abrasion est effectué avec au moins trois liquides de test différents appartenant aux groupes définis ci-dessus et **en ce que** lesdits au moins trois liquides de test différents appartiennent de préférence à trois groupes différents de liquides de test et/ou deux liquides de test sont des solutions alcooliques aqueuses.

7. Procédé selon les revendications 1 à 5, **caractérisé en ce qu'**au moins deux ou trois liquides de test différents appartiennent à des groupes différents de liquides de test.

8. Procédé selon l'une quelconque des revendications précédentes, **caractérisé en ce que** le textile plat est une structure plane telle qu'un tissu, un tricot, une maille, un tressage, un tricot cousu, un non-tissé et un feutre d'extension limitée, et en particulier un tissu de coton.

9. Procédé selon l'une des revendications précédentes, **caractérisé en ce que** les documents de sécurité ou les supports de données sont personnalisés par impression de la surface par transfert thermique ou par sublimation thermique ou par utilisation d'une encre spéciale à jet d'encre qui diffuse dans la couche supérieure et ou la surface du document de sécurité ou du support de données est en plastique, en papier, et en particulier en polycarbonate, PET, EVA ou PVC.

10. Ensemble de liquides de test pour la mise en œuvre de la vérification de la résistance des documents de sécurité et des supports de données aux agents désinfectants dans un procédé selon l'une quelconque des revendications 1 à 9, ledit ensemble étant constitué d'au moins deux liquides de test différents choisis dans les groupes des solutions alcooliques aqueuses, des liquides contenant des composés hydrophobes et des solutions contenant des biocides.
